# EUROPEAN PATENT SPECIFICATION

(11) **EP 1 867 238 B1**
(45) Date of publication and mention of the grant of the patent: **10.12.2014**
(21) Application number: 06729437.1
(22) Date of filing: 17.03.2006
(51) Int. Cl.: A23K 1/00, A23K 1/16, A23K 1/18, A61K 35/74, C12N 1/20

(54) **FEEDING STUFF COMPOSITION COMPRISING PROLIFERATION PROMOTING AGENT FOR BIFIDUS BACTERIA AND USE OF THE SAME**
TIERFUTTERZUSAMMENSETZUNG MIT EINEM MITTEL ZUR FÖRDERUNG DES WACHSTUMS VON BIFIDUS-BAKTERIEN, UND VERWENDUNG DER ZUSAMMENSETZUNG
COMPOSITION D ALIMENTS POUR BETAIL COMPRENANT UN AGENT PROMOTEUR DE LA PROLIFERATION DES BACTERIES BIFIDUS ET SON UTILISATION

(30) Priority: 18.03.2005 JP 2005079168
(43) Date of publication of application: 19.12.2007
(73) Proprietor: Meiji Co., Ltd., Tokyo 136-8908 (JP); Meiji Feed Co., Ltd., Chiyoda-ku Tokyo 101-0041 (JP)
(72) Inventor: HAGAWA, Yoshihito, Nerima-ku Tokyo 178-0063 (JP); YODA, Nobuo, Tokyo 136-8908 (JP); KANBE, Michio, Tokyo 189-0003 (JP); ORIHASHI, Takenori, Fuchu-shi Tokyo 183-0031 (JP)
(74) Representative: Kramer - Barske - Schmidtchen
(86) International application number: PCT/JP2006/305448
(87) International publication number: WO 2006/098447

(56) References cited:
- WO-A1-03/016544
- JP-A- 10 108 672
- JP-A- 10 304 871
- JP-A- 2004 305 128
- ISAWA K ET AL: "ISOLATION AND IDENTIFICATION OF A NEW BIFIDOGENIC GROWTH STIMULATOR PRODUCED BY PROPIONIBACTERIUM FREUDENREICHII ET-3" 1 March 2002 (2002-03-01), BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, PAGE(S) 679 - 681 , XP008041283 ISSN: 0916-8451 * abstract * * page 681, column 1, paragraph 2 *
- FUMIAKI ABE ET AL: "EFFECT OF ADMINISTRATION OF BIFIDOBACTERIA AND LACTIC ACID BACTERIATO NEWBORN CALVES AND PIGLETS" JOURNAL OF DAIRY SCIENCE, AMERICAN DAIRY SCIENCE ASSOCIATION, SAVOY, IL, US, vol. 78, no. 12, 1 December 1995 (1995-12-01), pages 2838-2846, XP000580307 ISSN: 0022-0302
- YORIDA N.: 'Propionibacterium ni yoru Nyusei Hakkoubutsu o Haigo shita Tablet 'B.G.S.'' NIPPON NOGEI KAGAKU KAISHI vol. 76, no. 7, 2002, pages 640 - 641, XP003002214
- OKADA Y.: 'Propionibacterium ni yoru Nyusei hakkobutsu Seibun no DHNA no DSS Choen ni Taisuru Ko Ensho Sayo no Kento' DIGESTIVE ORGAN AND IMMUNOLOGY vol. 40, 2003, pages 58 - 60, XP003002215
- YORITA N.: 'Series 'Health Claim no Kagakuteki Konyo' -11- propionibacterium Sansei Busshitsu no Bifidobacterium Zoshoku Sokushin Sayo' INTERNATIONAL LIFE SCIENCE INSTITUTE OF JAPAN vol. 80, 2004, pages 5 - 13, XP003002216

## Description

### Technical Field

The present invention relates to a feed composition for use in reducing and suppressing diseases of a newborn calf, shortly after the birth. The feed composition used according to the present invention has an intestinal flora improvement activity and is usable for milk replacer and a mixed feed for calves. It is possible to create a flora which is abundant with *Bifidobacterium* by administering the feed composition to a newborn calf immediately after the birth of the newborn calf.

### Background Art

It is not easy to steadily promote a growth of a domestic animal even when the domestic animal is an adult, and the growth promotion of a newborn domestic animal that is inferior in physical capacity and delicate is difficult. For example, in the case of a newborn calf, factors inhibiting the growth include insufficient colostrum ingestion, inappropriate environment (poor hygiene), change in feed, change from maternal milk to artificial feed (milk replacer, starter, or the like), stresses caused by transportation and change in breeding environment, and the like.

In the intestine of a healthy animal, a certain number of resident intestine microbiota is present on the intestinal mucosa to prevent infection with various pathogens. On the other hand, a balance in an intestinal flora is disturbed by changes in feed and environment, stress, administration of antibiotics, and the like. For example, there is a report that either of *Lactobacillus* or *Bifidobacterium* is reduced in calves suffering from diarrhea, and an importance of the useful bacterium is pointed out.

The calf intestinal flora is rapidly formed after the birth. It is desirable that a normal flora which is abundant with *Bifidobacterium* is formed and is maintained through and after a weaning period. However, actually, there are many inhibitory factors which affect the flora in actuality. For example, the colostrum is replaced by milk replacer 4 to 7 days after the birth, and diarrhea tends to occur.

After that, calves are transported to breeding farms from their birth places.

Since calves have poor adaptability to a change in feed, an increase in feed amount, transportation, and environmental stress, the intestinal flora which is insufficient in stability is influenced.

Additionally, the intestinal flora is disturbed by a weakened immune system due to insufficient colostrum, stress due to transportation and environmental change for calf introduction, a change in feed, and the like to cause the diarrhea.

The diarrhea occurring during a suckling period causes a growth stagnation that is a cause of adverse effects on the subsequent growth.

Hereinafter, specific examples of representative factors which affect the calf intestinal flora are described. Firstly, hygiene of the environments in which calves are born vary place by place. In view of the change from colostrum to milk replacer during the day 4 to 7 after the birth, there are individual differences in a quality and a feed amount of colostrum, and in quality and feed amount of the milk replacer.

Secondly, since the calves are introduced into another breeding farm before the age of 2 weeks after the birth, the calves suffered from stress due to transfer (transportation) and environmental change. Additionally, the breeding farms into which the calves are introduced are different in environmental condition, hygiene, breeding method, and the like.

Thirdly, passive immunity (passive antibodies) of calves is reduced to half in 2 to 3 weeks after the birth. There are individual differences in the reduction in passive immunity. A half life of a calf transfering antibody of IgG is 16 to 32 days; IgM is 4 days; and IgA is 2.5 days (see Non-Patent Publication 1, for example). IgG contributes to immunity of the whole body and intestinal tract, IgM contributes to immunity of prevention of septicemia in newborn calves up to the age of 3 days. IgA contributes to immunity of the mucous surface (local immunity).

Calves start to create antibodies after 2 weeks from the birth to produce active immunity, and the active immunity production is subject to individual difference.

Diseases which are common in calves are digestive diseases of which a main symptom is diarrhea and respiratory diseases of which a main symptom is pneumonia. The calf passive antibodies disappear in 2 to 3 weeks, and it is after 4 weeks from the birth that calves start to create satisfactory antibodies. Infections of calves of shortly after the birth due to insufficient IgG include diarrheal diseases caused by *E. coli, rotavirus, coronavirus, cryptosporidia, salmonella,* and the like. In a calf group which has a high incidence of pneumonia, diarrhea and insufficient nutrition after the birth as well as a functional loss of T-cells at one month after the birth, which is important for full growth of lymphatic cells, are observed.

Since a sick rate of calves is remarkably high and a death rate thereof frequently exceeds 5%, the sick rate and the death rate weigh down the management. For example, there is a report that 166 out of introduced 300 dairy bulls aging from 5 to 14 days were diagnosed as simple diarrhea (see Non-Patent Publication 2, for example). There is a strong association between the disease, particularly diarrhea, and the death rate.

In view of the above-described circumstances, as an improvement stimulator of intestinal flora for calves, probiotics such as *Enterococcus faecalis, Enterococcus faecium, Clostridium butyricum, Bacillus subtilis*, *Bacillus cereus, Bifidobacterium thermophilium, Bifidobacterium pseudolongum,* and *Lactobalillus acidophilus* and the like and oligosaccharides are commercially available.

However, in the case of the probiotics, it is difficult that the administered probiotics to live in the intestine, and the oligosaccharides have a drawback that it is utilized by many bacteria other than the *Bifidobacterium.* Therefore, the improvement stimulator of intestinal flora that is capable of specifically increasing only the useful bacterium that can live in the calf intestine for about 2 weeks after the birth has not been found yet.

On the other hand, although Profec (registered trademark: milk whey fermented by *Propionibacterium freudenreichii* ET-3) which is one of milk whey fermented by *Propionibacterium* has been known (see Non-Patent Publication 3, for example), there are no findings which confirm an effect obtained by administering the fermented milk whey to newborn domestic animals such as calves. Furthermore, there is no report on the reduction in death rate of newborn domestic animals by preventing diarrhea through formation and establishment of *Bifidobacterium* flora in the intestine of newborn domestic animals.

Non-Patent Publication 1: Katsurou Hagiwara, Kachiku Shinryou, 47(7), 477 (2000)

Non-Patent Publication 2: Kazuo Suzuki et al., Kachiku Shinryou, No. 256, 19 to 25 (October, 1984)

Non-Patent Publication 3: Nobuo Yoda: ILSI, No. 80, 5 to 13 (2004)

JP 10-108 672 A discloses the preparation of a Bifidus factor nutrient food in stick form, which nutrient food comprises a spray-dried product of a fermented solution of Propionibacterium freudenreichii ATCC 6207 strain, which was preliminary neutralized and cultured in 10 w/v % skim milk medium, pH 6.0 at 30 °C for 72 hours, followed by sterilisation.

WO 03/016544 A1 relates to a composition containing 1,4-dihydroxy-2-naphthoic acid, wherein the composition is obtained by culturing a microorganism, like for example of the genus Propionibacterium, in particular Propionibacterium freudenreichii ATCC 6207 strain, in a composition containing milk whey.

K. Isawa et al., Biosci. Biotechnol. Biochem., 66(3), 679-681, 2002, discloses a study on the isolation and identification of a new bifidogenic growth stimulator produced by Propionibacterium freudenreichii ET-3. In particular, D3 discloses the cultivation of Propionibacterium freudenreichii ET-3 in a 10 % (w/v) whey medium at 30 °C for 72 hours, wherein a corresponding culture broth is obtained.

JP 10-304871 A discloses an edible high concentration culture of propionic acid bacterium, wherein the propionic acid bacterium is cultivated in a medium comprising milk whey.

A. Fumiaki et al., Journal of Dairy Science 78(1995), no. 12, 2838-2846 relates to a study on the effect of oral administration of bifidobacteria and lactic acid bacteria on newborn livestock.

### Disclosure of the Invention

### Problems to be Solved by the Invention

In view of the present state that the sick rate of newborn domestic animals is high, and that, for example, in the case of calves, the death rate frequently exceeds 5% to weigh down the dairy farming management, an object of the present invention is to decrease the death rate by decreasing the sick rate of newborn calves, and to breed calves healthy.

More specifically, an object of the present invention is to rapidly and actively create *Bifidobacterium* flora in the course of formation of a stable intestinal flora after the birth of a newborn calf. Since the newborn calf increases *Bifidobacterium* which are in conformity with physiology of the calf having the *Bifidobacterium* after the formation of stable intestinal flora, establishment of grown *Bifidobacterium* is easy after the formation of stable intestinal flora. However, there is no example of a great increase of *Bifidobacterium* which is achieved by actively forming a favorable intestinal flora after the birth and before natural formation of the intestinal flora.

### Means for Solving the Problems

The present invention has been accomplished for the purpose of attaining the above object. The inventors of the present invention intended to actively form active *Bifidobacterium* flora after the birth of newborn calves and before natural intestinal flora formation, namely, they intended to form and establish the favorable intestinal flora firstly after the birth.

Although the intestine of a calf at the birth is in an antiseptic condition, bacteria are established in the intestine via colostrum of a dam and environmental factors (nipple, litter, water, and the like) to form a balanced intestinal flora in 3 weeks after the birth. During this important period, dominance of useful bacteria such as *Bifidobacterium* is remarkably effective for protecting the calf from diseases during such period. Also, there is a report that the useful bacteria are reduced by aging. The inventors of the present invention have noted the possibility of delaying such bacteria reduction by establishing the dominance of useful bacteria until 3 weeks, more preferably 2 weeks, after the birth and carried out screening on various substances.

The inventors of the present invention carried out screening on fermented milk whey obtained by *Propionibacterium freudenreichii* ET-3 as components which are selected from a tremendous number of substances and can form an intestinal flora which is abundant with *Bifidobacterium* at an earliest possible period after the birth of a newborn calf and made further studies to complete the present invention.

Namely, the present invention provides solving means for healthily breeding of newborn calves by forming and establishing *Bifidobacterium* flora in the intestine of new born calves with administration of fermented milk whey produced by *Propionibacterium freudenreichii* ET-3.

### Effect of the Invention

By oral administration of a feed composition used according to the present invention to a newborn calf, it is possible to form and further establish a useful *Bifidobacterium* flora in the intestine of the newborn calf: namely, it is possible to artificially form and establish the useful flora, at time when an intestinal flora has not been formed. Furthermore, as a result, it is possible to reduce and suppress various diseases including diarrhea of newborn calves to largely reduce a death rate.

### Brief Description of the Drawings

[Fig. 1] Number of *Bifidobacterium* in 1 g of excrement.
[Fig. 2] Proportion of *Bifidobacterium* in a total number of bacteria.

### Best Mode for Carrying out the Invention

Hereinafter, the present invention will be described in detail.

In the specification, parts, percentages, and proportions which are defined by mass have the same meanings as those defined by weight.

As *Propionibacterium* to be used in the present invention, the BGS producing probiotic *Propionibacterium freudenreichii* ET-3 (FERM BP-8115) is used.

Examples of a method for culturing the *Propionibacterium freudenreichii* ET-3 include a use of a culture medium containing a whey (a content of the whey in the culture medium may preferably be 1 to 20 mass%, more preferably 5 to 15 mass%), an aerobic or anaerobic culture in accordance with the ordinary method (for example, under a nitrogen gas pressurization (0.5 kg/cm²)), and the like. A liquid culture at 20°C to 40°C and at a pH level of 6 to 8 may ordinarily be preferred.

In the present invention, milk whey fermented by *Propionibacterium freudenreichii* ET-3 is used.

As the milk whey fermented by *Propionibacterium freudenreichii* ET-3, a culture product obtained by culturing *Propionibacterium freudenreichii* ET-3 in a culture medium containing milk whey (whey and/or a product thereof treated by protease), an isolated bacterium itself, a culture liquid (culture serum), a mixture thereof, and the like can be used widely. Other than the culture medium containing milk whey described above, a culture medium containing skim milk and the like may be used appropriately. A culture product obtained by culturing *Propionibacterium freudenreichii* ET-3 in the culture medium containing the skim milk, an isolated bacterium thenselves, a culture liquid (culture serum), a mixture thereof, and the like may be used in place of or in combination with the milk whey fermented by *Propionibacterium freudenreichii* ET-3.

For example, as one example of the milk whey fermented by *Propionibacterium freudenreichii* ET-3, it is possible to use milk whey fermented by *Propionibacterium freudenreichii* ET-3 (Profec) which is prepared by fermenting *Propionibacterium freudenreichii* ET-3 producing a Bifidogenic growth stimulator (BGS) by using whey powder reduction liquid. Although a content of the whey powder of the whey reduction liquid is not particularly limited, it may appropriately be from 1 to 25 wt%. In 100 g of the prepared Profec, 50 µg to 25 mg of 1,4-dihydroxy-2-naphthoic acid (DHNA) is contained.

As Profec to be used in the present invention, those obtained by the ordinary method, commercially available products, and the like may be used.

BGS which is the milk whey fermented by *Propionibacterium freudenreichii* ET-3 is 1,4-dihydroxy-2-naphthoic acid (DHNA) and 2-amino-3-carboxy-1,4-naphthoquinone (ACNQ). In these, 1,4-dihydroxy-2-naphthoic acid (DHNA) is a biosynthetic intermediate of vitamin K₂ (menaquinone) in microorganisms. These substances promote growth by effectively re-oxidizing NADH (nicotinamide adenine dinucleotide) generated in the course of energy metabolism of *Bifidobacterium.*

A dosage of the milk whey fermented by *Propionibacterium freudenreichii* ET-3 for a newborn calf is not particularly limited as long as the dosage stimulates formation and establishment of *Bifidobacterium* flora before natural formation of intestinal flora and after the birth of the newborn calf. It varies depending on a subject for administration, such as a type, a size, a symptom, and the like.

However, it is preferable to use the milk whey fermented by *Propionibacterium freudenreichii* ET-3 containing 1,4-dihydroxy-2-naphthoic acid and/or 2-amino-3-carboxy-1,4-naphthoquinone such amounts which stimulates formation and establishment of the *Bifidobacterium* flora. For example, in the case of 1,4-dihydroxy-2-naphthoic acid, the milk whey fermented by *Propionibacterium freudenreichii* ET-3 is administered in a day may preferably contain 1,4-dihydroxy-2-naphthoic acid in an amount of 2 to 600 µg, more preferably 6 to 240µg. The dosage of the milk whey fermented by *Propionibacterium freudenreichii* ET-3 may be divided into plural doses to be administered for plural times and not once, if necessary.

The milk whey fermented by *Propionibacterium freudenreichii* ET-3 prepared as described above is administrated as a feed composition which is mixed with an excipient (skim milk, starch, a cereal flour, sugar, and the like). The feed composition is orally administered to new born calves as a feed together with other feed components (for example, grass hay, grain, oilcake, bran, and the like). For example, it is possible to mix the feed composition used according to the present invention with milk replacer, a starter, drinking water, or the like to be administered. Since a dosage of the feed composition is remarkably small as described later in this specification, the administration does not impose any burden on the new born calves.

The dosage of the milk whey fermented by *Propionibacterium freudenreichii* ET-3 differs depending on the subject for the administration such as the type, the size, and the symptom and the like. For example, in the case of administering the milk whey fermented by *Propionibacterium freudenreichii* ET-3 alone to a calf, the dosage per day of the milk whey fermented by *Propionibacterium freudenreichii* ET-3 may be from 0.01 to 1200 g, preferably from 0.03 to 480 g. It is possible to administer the milk whey fermented by *Propionibacterium freudenreichii* ET-3 in an amount exceeding the range described above in some cases, and the dosage of the milk whey fermented by *Propionibacterium freudenreichii* ET-3 may be divided into plural doses to be administered for plural times and not once, if necessary.

In the case of administering a feed composition (hereinafter sometimes referred to as composition containing Profec) obtained by using Profec as the milk whey fermented by *Propionibacterium freudenreichii* ET-3; adding an excipient to Profec; and carrying out a treatment such as freeze-drying, if necessary, to a calf, from 0.04 to 10 g, preferably from 0.1 to 4 g of the feed composition per day may be mixed with a mill replacer and the like and be administered, although the dosage is varied depending on a proportion of the excipient, conditions of freeze-dried and the like, and the like. A remarkably small amount of the feed composition achieves an excellent effect. It is possible to administer the Profec-containing composition in an amount outside the above-specified range in some cases, and the dosage of the milk fermented by *Propionibacterium freudenreichii* ET-3 whey may be divided into plural doses to be administered for plural times and not once, if necessary. In the case of using a feed composition containing milk whey fermented by *Propionibacterium freudenreichii* ET-3 other than Profec, such feed composition may be administered according to the above-described dosage and the number of doses.

Although the newborn calves mean those up to and going through a weaning period, there is no problem when the feed composition of the present invention is administered to those that have passed the weaning period or to adult calves.

Although a period for administering the feed composition of the present invention is not particularly limited, it is preferable to administer the feed composition during a period in which a well-balanced intestinal flora is formed, a time period in which passive immunity is decreased, or a time with stress. More specifically, the feed composition of the present invention may preferably be administered not later than 3 weeks from the birth, more preferably not later than 2 weeks from the birth.

### Examples

Although examples of the present invention will hereinafter be described, the present invention is not limited to the examples.

In the examples, Profec was used as the milk whey fermented by *Propionibacterium freudenreichii* ET-3, and a feed composition (composition containing Profec) obtained by adding an excipient to Profec followed by freeze-drying was used. In 0.4 g of the composition containing Profec, 24 µg of 1,4-dihydroxy-2-naphthoic acid (DHNA) was contained.

### Example 1

### Experiment Schedule

To each of Holstein bull calves which were born in a contract farmer, a sufficient amount of colostrum was fed once immediately after the birth.

The calves were transferred to an experiment farm immediately after the colostrum feeding.

In order to eliminate influences caused by the differences in colostrum as much as possible, a colostrum replacer was administered to the calves from the second suckling to the day 3 after the birth.

After the day 4 after the birth, the colostrum replacer was replaced by a commercially available milk replacer, and the calves were divided into an experiment group and a control group, each consisting of 9 calves.

Each of the colostrum replacer and the commercially available milk replacer was administered in such a manner that 250 g of the colostrum replacer/milk replacer was dissolved into 1.8 L of warm water (45°C) and the thus-prepared replacer was administered twice a day, namely, in the morning and the evening.

To the experiment group, 0.2 g of the comprising milk whey fermented by *Propionibacterium freudenreichii* ET-3 (Profec) was mixed per suckling, i.e. 0.4 g of the Profec-containing composition was mixed and administered per day.

The experiment period was up to the age of 14 days after the birth.

### Experiment Period

(1) At the age of 3 days after the birth (before the start of experiment).
(2) At the age of 14 days after the birth (after the termination of experiment).

### Composition of colostrum replacer used for experiment

| | |
|---|---|
| Immunoglobulin-rich imported colostrum powder (skimmed) | 75% |
| Lactose | 6% |
| Vegetable oil | 19% |
| Total | 100% |

Ingredients: 57% of crude protein, 18% of crude fat, 15% of lactose; 15% of immune globulin

### Composition of commercially available milk replacer used for experiment

| | |
|---|---|
| Skim milk | 55% |
| Concentrated whey protein | 20% |
| Vegetable oil | 20% |
| Vitamin and mineral mixture | 5% |
| Total | 100% |

Ingredients: 24% of crude protein, 20% of crude fat, 110% of digestive nutrients total

### Intestinal Flora Improvement Experiment

The calves were divided into experiment groups described below to investigate changes in intestinal flora during each of the experiment periods. The experiment periods were as described above which are Experiment Periods (1) and (2). Bacteria which were subjects for the investigation were the following (A) to (F), and a number of bacterium in 1 g of excrement was counted.

Results obtained by the experiment are shown below, wherein all values show mean values.

### Experiment Group

Experiment group (Profec-fed group)
Control group

### Investigated Bacteria

(A) *Bifidobacterium*
(B) *Lactobacillus*
(C) *Enterobacteriaceae*
(D) *Bacteroidaceae*
(E) *Clostridium, Lecithinase*(+)
(F) *Clostridium, Lecithinase*(*-*)
(G) Total number of bacterium

### Results

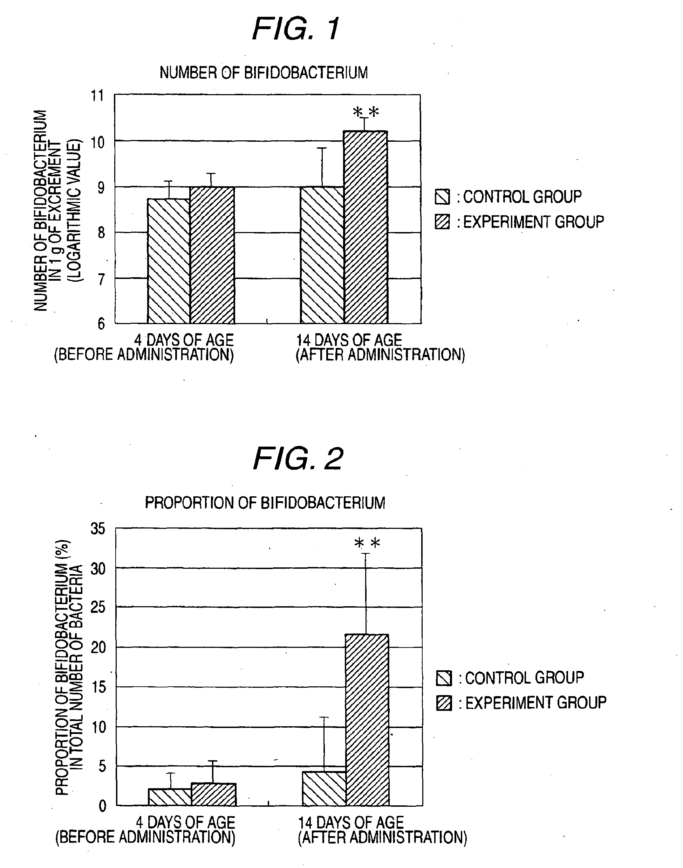

The results are shown in Tables 1 to 3 and Figs. 1 and 2. Data in each of tables are logarithmic bacterium number in 1 g of excrement, and a detection rate (%) is indicated in brackets. A significant difference between the experiment group and the control group at the age of 14 days was examined (**: p<0.01), (X: 3 days of age (before administration)), (Y: 14 days of age (after administration)).

**Table 1**

| | Experiment Group | | Control Group | |
|---|---|---|---|---|
| | X | Y | X | Y |
| (A) | 8.96±0.47 (100) | 10.19±0.30** (100) | 8.71±0.42 (100) | 8.98±0.95 (100) |
| (B) | 7.99±1.00 (100) | 8.37±0.68 (100) | 7.83±0.87 (100) | 8.20±0.89 (100) |
| (C) | 9.68±0.20 (100) | 9.06±0.29** (100) | 9.71±0.24 (100) | 9.14±0.47** (100) |
| (D) | 10.32±0.39 (100) | 10.75±0.20** (100) | 10.29±0.33 (100) | 10.45±0.53 (100) |
| (E) | 5.90±0.84 (100) | 3.69±0.86** (22) | 5.53±2.02 (89) | 4.62±2.08 (78) |
| (F) | 8.06±0.53 (78) | 8.45±0.21 (22) | 8.38±0.67 (100) | 8.47±0.34 (78) |
| (G) | 10.57±0.27 n=9 | 10.92±0.19** n=9 | 10.52±0.27 n=9 | 10.63±0.50 n=9 |

**Table 2**

| Number of *Bifidobacterium* | Mean value of number of bacterium | | Deviation | |
|---|---|---|---|---|
| | Control group | Experiment group | Control group | Experiment group |
| 4 days of age (before administration) | 8.71 | 8.96 | 0.42 | 0.47 |
| 14 days of age (after administration) | 8.98 | 10.19** | 0.95 | 0.30 |

**Table 3**

| Proportion of *Bifidobacterium* | % | | Deviation | |
|---|---|---|---|---|
| | Control group | Experiment group | Control group | Experiment group |
| 4 days of age (before administration) | 2.1 | 2.9 | 2.35 | 2.94 |
| 14 days of age (after administration) | 4.2 | 21.4** | 7.09 | 11.16 |

As is apparent from the above results, in the experiment group (0.4 g of Profec was administered per day for 10 days), *Bifidobacterium* in excrement significantly increased (from 8.96 to 10.19 Log10/g excrement), and the proportion in the total number of bacteria remarkably increased (from 2.9% to 21.4%). On the other hand, a detection rate of lecithinase (+) *Clostridium* remarkably decreased (from 100% to 22%). As described above, it was confirmed that Profec specifically increases *Bifidobacterium* in the intestine during the intestinal flora formation period after the birth and is effective for improving the intestinal flora through the in-vivo experiment using actual living calves.

### Industrial Applicability

By administering the feed composition used according to the present invention to new born calves, it is possible to form a useful *Bifidobacterium* flora in the intestine of newborn calves when an intestinal flora is not formed yet and further to establish the formed *Bifidobacterium* flora, namely, to artificially form and establish the useful flora. Consequently, it is possible to reduce and suppress diseases such as diarrhea of the newborn calves to decrease a death rate.

## Claims

1. A feed composition comprising milk whey fermented by *Propionibacterium freudenreichii* ET-3 (FERM BP-8115) for use in reducing and suppressing diarrhea of a newborn calf by orally administering the feed composition to the calf, wherein the calf has an age of up to 14 days.

2. The feed pomposition for the use according to claim 1, wherein the method for fermenting the *Propionibacterium* includes the use of a culture medium containing 1 to 20 mass% of milk whey.

3. The feed composition for the use according to claim 1 or 2, wherein the feed composition is administered in an amount in which the feed composition comprises 1,4-dihydroxy-2-naphthoic acid in a range of 2 to 600 µg per day.

4. The feed composition for the use according to claim 3, wherein the administered content of 1,4-dihydroxy-2-naphthoic acid in the feed composition is in a range of 6 to 240 µg per day.

5. The feed composition for the use according to claim 1 or 2, wherein the feed composition is a feed composition obtainable by using Profec^{®} as the milk whey fermented by *Propionibacterium freudenreichii* ET-3 and adding an excipient to Profec^{®} and is administered in an amount of 0.04 to 10 g per day.

6. The feed composition for the use according to claim 5, wherein the feed composition is administered in an amount of 0.1 to 4 g per day.

## Patentansprüche

1. Futterzusammensetzung, die Molke umfasst, die durch *Propionibacterium freudenreichii* ET-3 (FERM BP-8115) fermentiert worden ist, zur Verwendung bei der Verminderung und Unterdrückung einer Diarrhö eines neugeborenen Kalbs durch orales Verabreichen der Futterzusammensetzung an das Kalb, wobei das Kalb bis zu 14 Tage alt ist.

2. Futterzusammensetzung zur Verwendung nach Anspruch 1, wobei das Verfahren zum Fermentieren des *Propionibacteriums* die Verwendung eines Kulturmediums umfasst, das 1 bis 20 Massen-% Molke umfasst.

3. Futterzusammensetzung zur Verwendung nach Anspruch 1 oder 2, wobei die Futterzusammensetzung in einer Menge verabreicht wird, so dass die Futterzusammensetzung 1,4-Dihydroxy-2-naphthoesäure in einem Bereich von 2 bis 600 µg pro Tag umfasst.

4. Futterzusammensetzung zur Verwendung nach Anspruch 3, wobei der verabreichte Gehalt von 1,4-Dihydroxy-2-naphthoesäure in der Futterzusammensetzung in einem Bereich von 6 bis 240 µg pro Tag liegt.

5. Futterzusammensetzung zur Verwendung nach Anspruch 1 oder 2, wobei die Futterzusammensetzung eine Futterzusammensetzung ist, die durch die Verwendung von Profec^{®} als Molke, die durch *Propionibacterium freudenreichii* ET-3 fermentiert wird, und Zusetzen eines Hilfsstoffs zu Profec^{®} erhältlich ist und in einer Menge von 0,04 bis 10 g pro Tag verabreicht wird.

6. Futterzusammensetzung zur Verwendung nach Anspruch 5, wobei die Futterzusammensetzung in einer Menge von 0,1 bis 4 g pro Tag verabreicht wird.

## Revendications

1. Composition d'aliments comprenant du lactosérum de lait fermenté par Propionibacterium freudenreichii ET-3 (FERM BP-8115) pour une utilisation dans la réduction et la suppression de la diarrhée d'un veau nouveau-né par administration orale de la composition d'aliments au veau, où le veau présente un âge jusqu'à 14 jours.

2. Composition d'aliments pour l'utilisation selon la revendication 1, où la méthode de fermentation de Propionibacterium comprend l'utilisation d'un milieu de culture comprenant 1 à 20 % en masse de lactosérum de lait.

3. Composition d'aliments pour l'utilisation selon la revendication 1 ou 2, où la composition d'aliments est administrée en une quantité selon laquelle la composition d'aliments comprend de l'acide 1,4-dihydroxy-2-naphtoïque dans une plage de 2 à 600 µg par jour.

4. Composition d'aliments pour l'utilisation selon la revendication 3, où le contenu administré d'acide 1,4-dihydroxy-2-naphtoïque dans la composition d'aliments est dans une plage de 6 à 240 µg par jour.

5. Composition d'aliments pour l'utilisation selon la revendication 1 ou 2, où la composition d'aliments est une composition d'aliments pouvant être obtenue par une utilisation de Profec® en tant que le lactosérum de lait fermenté par Propionibacterium freudenreichii ET-3 et un ajout d'un excipient à Profec® et est administrée en une quantité de 0,04 à 10 g par jour.

6. Composition d'aliments l'utilisation selon la revendication 5, où la composition d'aliments est administrée en une quantité de 0,1 à 4 g par jour.
